# EUROPEAN PATENT SPECIFICATION

(11) **EP 3 739 432 B1**
(45) Date of publication and mention of the grant of the patent: **26.08.2026**
(21) Application number: 20174619.5
(22) Date of filing: 14.05.2020
(51) Int. Cl.: G06F 3/01, G02B 27/00, G02B 27/01

(54) **METHOD AND SYSTEM FOR DWELL-LESS, HANDS-FREE INTERACTION WITH A SELECTABLE OBJECT**
VERFAHREN UND SYSTEM ZUR HALTEZEITFREIEN, HANDFREIEN INTERAKTION MIT EINEM AUSWÄHLBAREN OBJEKT
PROCÉDÉ ET SYSTÈME D'INTERACTION MAINS LIBRES SANS MAINTIEN AVEC UN OBJET SÉLECTIONNABLE

(30) Priority: 15.05.2019 SE 1950580
(43) Date of publication of application: 18.11.2020
(73) Proprietor: Tobii AB, 182 17 Danderyd (SE)
(72) Inventor: Ratcliff, Andrew, 182 17 Danderyd (SE)
(74) Representative: Novagraaf Group

(56) References cited:
- EP-A1- 2 709 060
- JP-A- 2010 262 189
- US-A1- 2017 131 764

## Description

### Technical field

The present disclosure relates generally to methods, systems and devices for dwell-less, hands-free interaction, especially in augmented reality, AR, or virtual reality, VR, environments.

### Background

Several different eye-tracking systems are known in the art. Such systems may, for example, be employed to allow a user to indicate a location at a computer display or in an augmented reality, AR/ virtual reality, VR environment by looking at that point. The eye-tracking system may capture images of the user's face, and then employ image processing to extract key features from the user's face, such as a pupil center and glints from illuminators illuminating the user's face. The extracted features may then be employed to determine where at the display the user is looking. Furthermore, the user may interact with objects (e.g. buttons, switches, keys, UI elements etc.) on the computer display or in the AR/VR environment by resting or dwelling the eyes on the object until it activates. Dwelling is the act of fixing the eyes on part of the computer display or the AR/VR environment, and keeping the eyes there for a specific amount of time. The amount of time is called dwell time.

Interaction by means of dwelling is useful in many situations where the user's hands cannot be used, e.g. surgeons, engineers, disabled people etc., but the user still needs to interact with **UI** elements on a computer display or in an ARNR environment. However, in cases where extended interaction with several objects is required, repeated dwelling becomes both straining for the eyes of the user as well as time-consuming and tedious since the user cannot influence the dwell time. US20170131764A1 relates to allowing a person to exert control in real space as well as in virtual spaces. Eye tracking is employed to determine the direction of the viewer's gaze. Eye vergence can then be used to as a triggering mechanism to initiate responses stored in association with objects in the field of view. Additionally, eye vergence can be used to provide a continuous control. EP2709060A1 shows a system for determining the gaze endpoint of a subject. By using the vergence to calculate the intersection of the gaze direction of the eyes of the subject there can be determined the gaze endpoint. This gaze endpoint can then be used to determine the object the user is gazing at. JP2010262189A relates to multifocal electronic spectacles with a variable focus lens.

Consequently, there exists a need for improvement when it comes to interacting with selectable objects on a computer display or in an AR/VR environment using eye-tracking.

### Summary

The invention is set out in the independent claims. Preferred embodiments are defined by the dependent claims.

It is an object of the invention to address at least some of the problems and issues outlined above. An object of embodiments of the invention is to provide a device and a method which improves the eye-tracking experience for a user with respect to interaction with selectable objects by reducing the dwell time, both on a computer display, and in an AR/VR environment displayed for instance by means of a head-mounted display (HMD). It may be possible to achieve these objects, and others, by using methods, devices and computer programs as defined in the attached independent claims.

According to a first aspect of the present invention, there is provided a method for interacting with a selectable object displayed by means of a displaying device, the method comprising the steps of: obtaining, by means of an eye tracker, a gaze convergence distance and a gaze direction of a user, the gaze direction lying in a field of view defined by the displaying device, wherein the gaze convergence distance is defined as a distance from the user to a convergence point; determining whether the gaze direction coincides with the selectable object; and if so, detecting a change in the gaze convergence distance; and if the detected change in the gaze convergence distance exceeds a predetermined threshold value, interacting with the selectable object, wherein interaction with the selectable object is performed only if during detection of the change in the convergence distance: a predetermined change of the gaze direction is detected.

By using the gaze convergence distance and gaze direction of the user to interact with selectable objects, a quicker method for hands-free and dwell-less interaction with objects is achieved. The user may actively select the object by changing the vergence of the eyes, i.e. refocusing the gaze to a different depth, instead of having to dwell on the object for a certain period of time. The predetermined change of the gaze direction may correspond to the user moving his/her gaze in a predetermined direction or predetermined angle in relation to the selectable object. The additional requirement on the gaze direction increases the robustness of the system in that the user must perform a predetermined action in order to effect interaction, which will prevent accidental interaction.

In a preferred embodiment, the step of detecting further comprises the step of: obtaining an object distance, indicating the distance between the user and the selectable object; and a trigger distance, wherein the difference between the object distance and the trigger distance corresponds to the predetermined threshold value. Thus, the endpoints of the predetermined threshold value for the gaze convergence distance are defined in relation to the selectable object and may be used in different applications to aid the user.

In a further preferred embodiment, the step of detecting further comprises priming the selectable object for interaction when the gaze direction coincides with the selectable object and the gaze convergence distance is within a predetermined range comprising the object distance, and the step of interacting further comprises interacting with the primed selectable object. In this way, a solid and stable method for reliably effecting hands-free interaction is achieved which is resilient to the noise usually associated with eye tracking. Further, noise is reduced by requiring the user to not only look in the direction of the selectable object, but also focus the gaze within a predetermined range from the selectable object, i.e. nearer or beyond, before the object may be selected.

In an advantageous embodiment, priming the selectable object comprises changing a displayed appearance of the selectable object. The displayed appearance of the selectable object may be changed for instance to a so called "ghost" which makes the primed selectable object stand out from other selectable objects. Another alternative is to change the size, color and/or sharpness of the selectable object and/or the distance from the user. By displaying the ghost at a distance corresponding to the threshold for interaction, the user is aided in refocusing the gaze to the extent required for effecting interaction.

In an alternative embodiment, the method further comprises displaying a representation of the gaze direction of the user and/or a trigger distance corresponding to the predetermined threshold value. Preferably, the representation of the gaze direction/trigger distance comprises an alphanumeric character, a logographic character, a geometrical figure and/or a symbol or a representation of the selectable object. For instance, the gaze direction/trigger distance may be represented by a point, a ring or a reticle (crosshairs) to further aid the user during positioning of the gaze. In one embodiment, displaying of the gaze direction/trigger distance may be initiated only once the user has fixed the gaze on a selectable object, i.e. when the gaze direction coincides with the selectable object in conjunction with priming a selectable object for interaction.

In an advantageous embodiment, the method further comprises changing a visual appearance of the representation of the gaze direction/trigger distance in dependence of the convergence distance of the user. In one embodiment, the representation of the gaze direction/trigger distance is displayed at a different depth of view than the selectable object. Preferably, the representation of the gaze direction/trigger distance is displayed at a distance from the selectable object corresponding to the predetermined threshold value. Changing the appearance as well as displaying the gaze direction at a different depth of view (i.e. distance from the user) further aids the user in discerning the gaze direction and visualizing the trigger distance for effecting interaction.

In a further preferred embodiment, interaction with the selectable object is performed only if during detection of the change in the convergence distance: the gaze direction remains coinciding with the selectable object, the gaze direction coincides with a second object displayed by the displaying device and different from the selectable object.

In an alternative embodiment, the step of obtaining the gaze convergence distance and the gaze direction of the user comprises acquiring gaze data for each of the eyes of the user by means of an eye tracker and calculating the gaze convergence distance and the gaze direction based on the acquired gaze data. The gaze data comprises at least gaze direction and pupil position or gaze ray origin of each of the eyes of the user to be used in the calculation of the gaze direction and the gaze convergence distance. Generally, the gaze data including the gaze direction and the gaze convergence distance may be indirectly obtained from an external device such as an eye tracker, or directly acquired and calculated as part of the method.

In a preferred embodiment, the gaze direction is based on a combined gaze direction (CGD) obtained by combining the gaze directions of the left eye and the right eye of the user. By combining the left and right eye's gaze direction signals reliability and stability is achieved. The new "combined" direction vector will remain the same whether the users looks near or far.

In an advantageous embodiment, the convergence distance is a function of an interpupillary distance (IPD) of the user in relation to the interocular distance (IOD) and eyeball radius, based on the acquired pupil position or gaze ray origins of the left eye and the right eye. By comparing the raw positions for each eye (Pupil In Sensor), a robust indication of gaze convergence distance is achieved.

In an alternative embodiment, the selectable object comprises an actuatable user interface (UI) element, such as a virtual switch, button, object and/or key. The UI element may be any actuatable object that the user may interact with in the environment displayed by means of the displaying device.

In a further preferred embodiment, the actuatable UI element is connected to a real electronically operated switch such that interaction with the UI element causes actuation of the real electronically operated switch. Any real world electronically operated "connected" switch could be actuated by means of interaction with the selectable object. In the context of e.g. an AR environment, the term connected may be interpreted as accessible by the same software as is used to detect the convergence distance change - it could be connected via Bluetooth, Wi-Fi etc. Examples include TV, microwave ovens, heating, light switch, audio mute switch, factory machinery or medical equipment etc. For instance, a busy flight controller in an airport tower could easily open up a voice channel to the airplane he/she is looking at.

In a second aspect of the present invention, there is provided a system for displaying and interacting with a selectable object, the system comprising: a displaying device arranged to display one or more selectable objects; processing circuitry; at least one eye tracker configured to acquire gaze data for both of the eyes of a user, and a memory, said memory containing instructions executable by said processing circuitry, whereby the system is operative for: obtaining by means of the eye tracker, a gaze convergence distance and a gaze direction of a user, the gaze direction lying in a field of view defined by the displaying device, wherein the gaze convergence distance is defined as a distance from the user to a convergence point, wherein the system is operative for calculating the gaze convergence distance and the gaze direction based on the acquired gaze data; determining whether the gaze direction coincides with the selectable object; and if so, detecting a change in the gaze convergence distance; and if the detected change in the gaze convergence distance exceeds a predetermined threshold value, interacting with the selectable object, wherein interaction with the selectable object is performed only if during detection of the change in the convergence distance: a predetermined change of the gaze direction is detected.

In further preferred embodiments, the system is further operative for performing the method according to the first aspect.

In an advantageous embodiment, the displaying device is a head-mounted display (HMD) and the selectable object is projected at a predetermined distance by the HMD.

In an alternative embodiment, the displaying device is a physical display and the selectable object is displayed at the physical display.

In a third aspect of the present invention, there is provided a computer program comprising computer readable code means to be run in a system for displaying and interacting with a selectable object, which computer readable code means when run in the system causes the system to perform the following steps: obtaining, by means of an eye tracker, a gaze convergence distance and a gaze direction of a user, the gaze direction lying in a field of view defined by the displaying device, wherein the gaze convergence distance is defined as a distance from the user to a convergence point; determining whether the gaze direction coincides with the selectable object; and if so, detecting a change in the gaze convergence distance; and if the detected change in the gaze convergence distance exceeds a predetermined threshold value, interacting with the selectable object, wherein interaction with the selectable object is performed only if during detection of the change in the convergence distance: a predetermined change of the gaze direction is detected.

In alternative embodiments, the computer readable code means when run in the system further causes the system to perform the method according to the first aspect.

In a fourth aspect of the present invention, there is provided a carrier containing the computer program according to the third aspect, wherein the carrier is one of an electronic signal, an optical signal, a radio signal or a computer readable storage medium.

Further possible features and benefits of this solution will become apparent from the detailed description below.

### Brief description of drawings

The solution will now be described in more detail by means of exemplary embodiments and with reference to the accompanying drawings, in which:
Figs. 1A and 1B are top down views in a horizontal plane illustrating a gaze direction of a user in relation to a reference point at two different convergence distances;
Fig. 2 is a schematic view of a method for interacting with a selectable object according to one embodiment of the present disclosure;
Figs. 3A-3C are perspective views of a user turning a switch ON and OFF in accordance with one embodiment of the present disclosure;
Fig. 4 is a flow chart illustrating interaction with a selectable object according to one embodiment of the present disclosure;
Figs. 5A and 5B are perspective views of a user typing letters in accordance with one embodiment of the present disclosure;
Figs. 5C and 5D illustrate views from the perspective of the user typing letters in accordance with one embodiment of the present disclosure;
Figs. 6A and 6B are perspective views showing different visual representations of features according to a further embodiment of the present disclosure;
Figs. 7A and 7B illustrate views in accordance with the embodiment of Figs. 6A and 6B from the perspective of the user in combination with top-down horizontal views;
Figs. 8A-8E illustrate views from the perspective of the user in combination with top-down horizontal views of features according to a further embodiment of the present disclosure;
Fig. 9 shows a block schematic of a system according to an embodiment of the present disclosure; and
Figs. 10A and 10B illustrate views of displaying devices according to different embodiments of the present disclosure.

### Detailed description

Briefly described, a method for interacting with a selectable object displayed by a displaying device is provided, which facilitates interaction with objects to improve the eye-tracking experience of the user. By using methods, devices and computer programs according to the present disclosure, the experience of eye-tracking for the user can be improved by enabling faster and more robust interaction with selectable objects, for instance on a screen or display, or in a augmented reality, AR, or virtual reality, VR, environment.

In the context of the present disclosure, the term 'selectable object' should be interpreted broadly as encompassing any type of displayed object that a user may interact with in order to perform a function or carry out an action.

In the context of the present disclosure, the term 'interaction' should be interpreted broadly as encompassing any type of action that may be carried out by a user on a selectable object displayed to the user, including but not limited to selection, querying, actuation, collection.

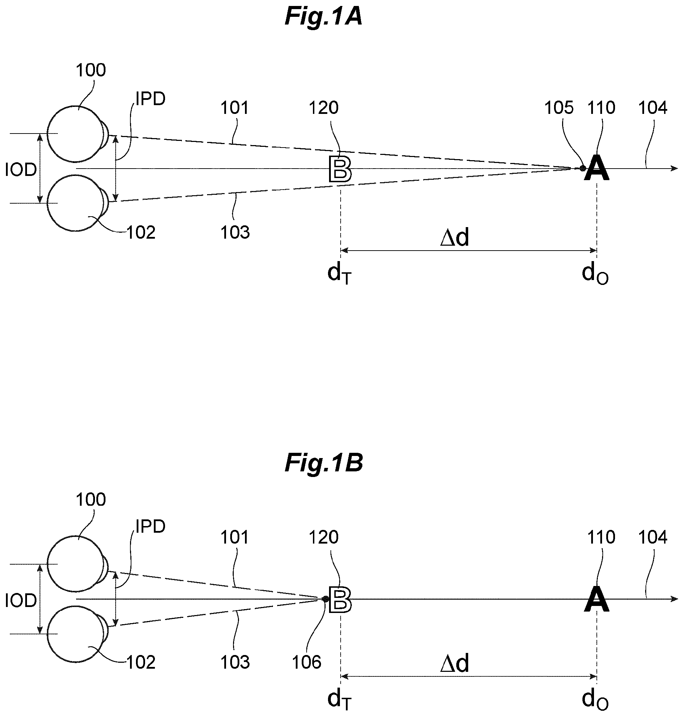

Figs. 1A and 1B illustrate schematically the underlying principle of the present invention. Shown here are the eyes of a user in a top down view in a horizontal plane with the dashed lines representing the gaze rays 101, 103 or gaze directions of the left eye 100 and right eye 102, respectively. In order to determine in which direction the user is looking, or more precisely what the user is looking at, the gaze directions of left and right eye 100, 102 may then be combined to a combined gaze direction (CGD) vector 104, represented in Figs. 1A and 1B by the solid arrow between the left and right eye 100, 102. In the context of displaying devices such as screens and displays of computers, tablets and smartphones or head-mounted displays such as virtual reality, VR, headsets, augmented reality, AR, headsets and/or mixed reality, MR, headsets, the CGD vector 104 may be translated to a gaze direction lying in a field of view defined by the displaying device 10. For instance, on a computer screen, the gaze direction is taken as the point of intersection between the CGD vector 104 and the plane of the screen which indicates what (object) the user is looking at.

In Fig. 1A, the user looks at a displayed selectable object 110, here represented by the letter 'A', and focuses the gaze of the left and right eyes 100, 102 to a first depth or convergence distance indicated by the intersection of the gaze rays 101, 103 at convergence point 105 near the letter 'A'. In Fig. 1B, the user has focused the gaze to a second depth or convergence distance smaller than the first convergence distance, which is shown by the gaze rays intersecting at a convergence point 106 closer to the user. As will be explained further in detail below, the letter 'B' is a representation 120 of the user's gaze direction and/or a trigger distance d_{T} and is shown at a position corresponding to the second depth or convergence distance. However, the combined gaze direction illustrated by the CGD vector 104 remains unchanged regardless whether the user looks near or far.

In accordance with one or more embodiments of the present disclosure, the convergence distance may be calculated based on the gaze rays 101, 103, also called visual axes. Each eye 100, 102 can be associated with a visual axis 101, 103, and the convergence point 105, 106 can be determined as the point of intersection of the axes. A depth can then be calculated from the user to the convergence point. The gaze convergence distance can be calculated as a depth/distance from a first eye 100 to the convergence point 105, 106, can be calculated as a depth/distance from a second eye 102 to the convergence point 105, 106 or by calculating a depth/distance from a normal between the first eye 100 and the second eye 102 to the convergence point 105, 106.

Alternatively, the convergence distance may be calculated based on interocular distance, IOD, and interpupillary distance, IPD, according to one or more embodiments of the present disclosure. In one embodiment, the gaze convergence distance is obtained using IOD, indicating a distance between the eyes of the user, and IPD, indicating a distance between the pupils of the user, and a predetermined function.

In one example, the predetermined function is implemented in the form of a look-up table or other data structure capable to identify a particular convergence distance using a pair of IOD and IPD values. The look-up table or other data structure may be built up or created by monitoring measured IOD and IPD values whilst allowing a user to focus on objects at different depths.

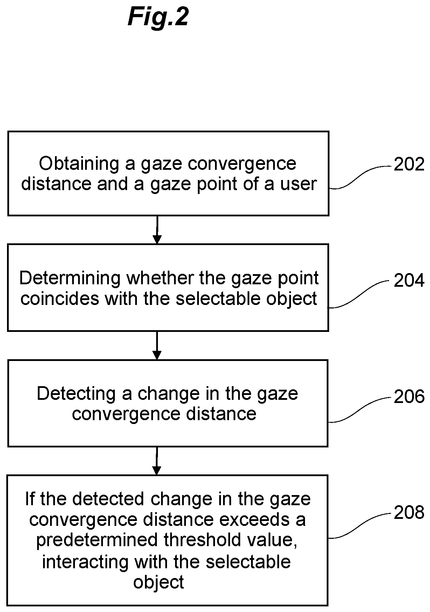

Fig. 2, in conjunction with Figs. 1A and 1B, shows an embodiment of a method for interacting with a selectable object 110 displayed by a displaying device 10. The method may in some embodiments be performed by a system 900 comprising the displaying device 10, and in some embodiments, the method may be performed elsewhere using data obtained from an eye tracker and the displaying device 10, for example in a cloud environment to which the eye tracker and the displaying device 10 is operatively connected.

The method comprises obtaining 202 a gaze convergence distance and a gaze direction of a user, the gaze direction lying in a field of view defined by the displaying device 10. As mentioned above, the eye data from which the gaze convergence distance and a gaze direction is obtained may be acquired by an external eye tracker communicating with or operatively connected to the displaying device 10, or an eye tracker integrated with the displaying device 10.

The method further comprises determining 204 whether the gaze direction coincides with the selectable object 110. If that is the case, the method further comprises detecting 206 a change in the gaze convergence distance, and if the detected change in the gaze convergence distance exceeds a predetermined threshold value, interacting 208 with the selectable object 110.

In both Figs. 1A and 1B there is indicated an object distance d_{O} which substantially corresponds to the distance between the user and the selectable object 110. Additionally, a trigger distance d_{T} is shown which substantially corresponds to the distance between the user and the depth to which the convergence distance has to travel in order to interact with the selectable object 110, wherein the difference Δd between the object distance d_{O} and the trigger distance d_{T} corresponds to the predetermined threshold value noted above. In Figs. 1A and 1B, the trigger distance d_{T} corresponds to a distance shorter than the object distance d_{O}. This implies that the user has to focus at a depth closer to the user than the object distance d_{O}. However, in some embodiments, the trigger distance d_{T} may be greater than the object distance d_{O}, i.e. further away from the user. In this case, the user has to focus at a depth further away from the object distance d_{O}.

Put in other words, as the user is looking at the selectable object 110 displayed by the displaying device 10, a change in the convergence distance, i.e. refocusing of the user's eyes 100, 102 from the object distance d_{O} to the trigger distance d_{T}, is detected and used as a trigger or selection signal to interact with the object 110 the user is looking at, but only if the detected change in the convergence distance exceeds the threshold value Δd. By means of the method according to the present disclosure, the user may interact with and select displayed objects 110 without having to dwell on the object 110 for an extended period of time.

In some embodiments, the method further comprises displaying a representation 120 of the gaze direction and/or the trigger distance d_{T}, also called a "ghost" or "trigger" in the following. The representation 120 of the gaze direction and/or trigger distance d_{T} as a trigger may include an alphanumeric character (letters and digits), a syllabic character (e.g. Korean Hangul), a logographic character (e.g. Chinese characters, Japanese Kanji), a geometrical figure such as a circle, a square, a triangle, a point, a cross, crosshairs, an arrow or any other symbol. As explained above with reference to Figs. 1A and 1B, the letter 'B' is one example of such a trigger or ghost. In some embodiments, the representation 120 of the gaze direction and/or trigger distance d_{T} as a ghost may be a visual representation 120 of the selectable object 110 which the user is looking at. In such cases, the ghost of the selectable object 110 may be displayed with a slightly changed visual appearance compared to the actual selectable object 110, for instance different in size, color, visual depth and/or sharpness. In some embodiments, both a ghost and a trigger may be displayed by the displaying device 10.

The ghost or trigger acts as a visual aid to the user to more clearly indicate what the user is looking at. In some embodiments, the ghost and/or trigger may be displayed at different depth of view than the selectable objects 110, for instance at a distance from the selectable object corresponding to the predetermined threshold value also called trigger distance d_{T}. In the latter case, the trigger will then further aid the user in indicating to which depth of view the gaze must be refocused, i.e. how much the convergence distance must be changed, in order to interact with the selectable object 110. In yet other embodiments, the visual appearance of the ghost and/or trigger may change, for instance gradually, as the convergence distance of the user changes, as will be further explained below.

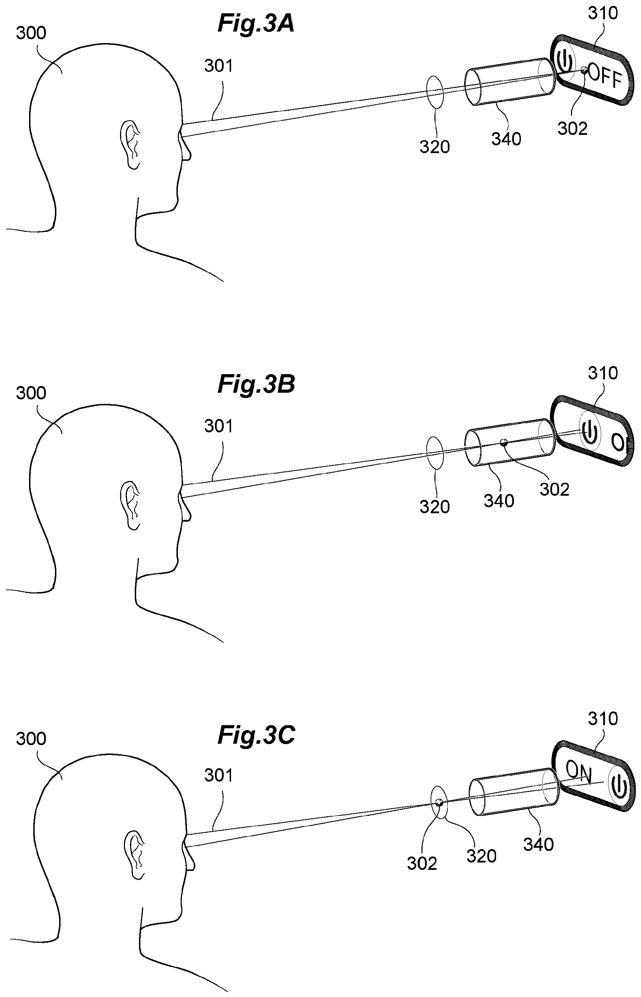

Turning now to Figs. 3A-3C, there is shown perspective views of a user 300 interacting with a selectable object 310 by means of the method according to the present disclosure. In Fig. 3A, the user 300 gazes at a selectable object 310 displayed by the displaying device 10, in the form of an ON/OFF switch 310 currently in OFF mode, as indicated by the gaze rays 301, i.e. the user's 300 gaze direction coincides with the ON/OFF switch 310. The convergence distance of the user 300 is represented by a convergence point 302 indicating that the user 300 currently focuses his gaze at the ON/OFF switch 310. Also, in Figs. 3A-3C the representation 320 of the gaze direction is displayed to the user 300 in the form of a circle acting as a trigger/visual aid as explained above.

In some embodiments, interaction with the selectable object 110; 310 is only performed if the gaze direction remains coinciding with the selectable object 110; 310 during detection of the change in the convergence distance. Put differently, the user 300 must keep looking at the selectable object 110; 310 as he refocuses his gaze to cause interaction with the selectable object 110; 310. This additional condition for effecting interaction with selectable objects 310 is illustrated in Figs. 3A-3C by means of a pipe or tube 340, which may or may not be displayed to the user 300. During refocusing of the gaze, the convergence point 302 may not breach the tube 340, otherwise the interaction is aborted. The length of the tube 340 is chosen to correspond to the predetermined threshold value Δd such that its proximal end is located at the trigger distance d_{T} mentioned above.

In alternative embodiments, the gaze direction is required to coincide with a second object displayed by the displaying device 10 and different from the selectable object 310. A predetermined change of the gaze direction must be detected during detection of the change in the convergence distance. For instance, the displaying device 10 may display a button at a predetermined position in the field of view, or the user 300 is required to move the gaze in a predetermined direction or pattern to carry out interaction.

In Fig. 3B, the convergence point 302 travels through the tube 340 towards the user 300 as he refocuses the gaze. At the same time, the ON/OFF switch 310 starts to toggle from OFF to ON, which further aids the user 300 in indicating that the interaction with the ON/OFF switch 310 is in progress.

In Fig. 3C, the convergence point 302 has reached the trigger circle 320 without breaching the wall of the tube 340 and the ON/OFF switch 310 is fully toggled to an ON state. The same procedure may now be repeated in order to turn the ON/OFF switch 310 back to the OFF state.

In order to avoid accidental interaction with objects, the present disclosure further provides a robust filtering method which reduces noise usually associated with eye tracking. The filtering comprises dividing the interaction with the selectable object 110 into two phases or steps. In a first step, the selectable object 110 is primed for interaction when the gaze direction coincides with the selectable object 110 and the gaze convergence distance exceeds a predetermined distance. In some embodiments, the predetermined distance corresponds to the object distance d_{O} described above in conjunction with Figs. 1A and 1B and/or the distance between the user and the selectable object 110. In other words, when the user gazes at a selectable object 110 and focuses the gaze at (or beyond) the selectable object 110, the selectable object 110 is primed for interaction. Priming indicates to the user that the selectable object 110 is ready for interaction. In some embodiments, priming the selectable object 110 comprises changing a displayed appearance of the selectable object 110. This may include displaying a ghost of the selectable object 110 as discussed above as well as changing the displayed appearance of the ghost.

In a second step, the primed selectable object 110 is selected when the detected change in the gaze convergence distance exceeds the predetermined threshold value Δd. In other words, in order to perform an interaction, the selectable object 110 must first be primed during the first step outlined above.

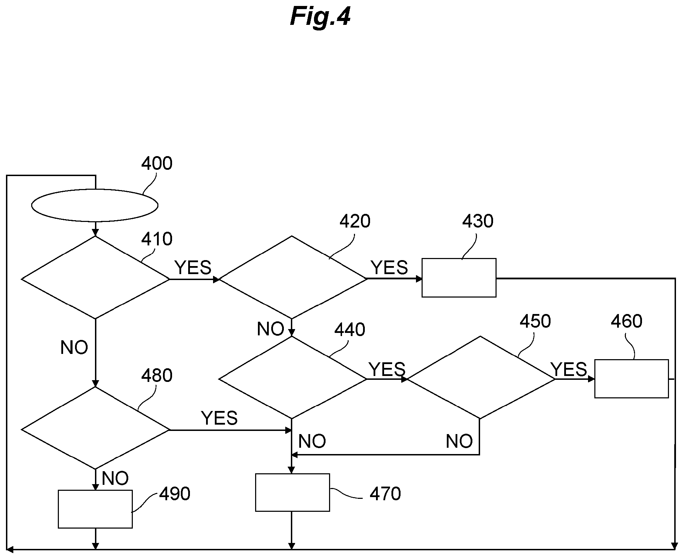

Fig. 4 illustrates a flow chart of an algorithm or filtering logic demonstrating this further embodiment of the method according to the present disclosure with respect to a selectable object 110. For simplicity, it is assumed in this example that the selectable object 110 is a button such as a key on a keyboard or an ON/OFF button. The button may be in one of two positions, DOWN indicating that the button is/has been selected and UP indicating that the button is not selected.

In an initial situation illustrated by the oval 400 at the top of the flow chart, a new gaze signal is received. In a first step 410, it is determined whether the gaze direction coincides with the button. If yes, in a subsequent step 420 it is determined whether the convergence distance is greater than the object distance d_{O}. If yes, the button is primed for interaction in step 430 and the algorithm returns to the initial stage 400 ready to receive a new gaze signal. In the case where the convergence distance is not greater than the object distance d_{O}, the algorithm proceeds to step 440 to determine whether a change in the convergence distance greater than the predetermined threshold value Δd has occurred, i.e. whether the convergence distance is less than the trigger distance d_{T} (or greater than the trigger distance d_{T} in the case of d_{T} > d_{D}). If yes, in a subsequent step 450 it is determined whether the button is primed, and if so the button is moved to the DOWN position in step 460 and the algorithm returns to the initial stage 400. Otherwise, if the convergence distance is not less than the trigger distance d_{T} (or not greater than the trigger distance d_{T} in the case of d_{T} > d_{D}) or if the button is not primed (i.e. either of steps 440 or 450 returns a negative response), the button is moved to the UP position in step 470 and the algorithm returns to the initial stage 400.

Going back to the case where the gaze direction does not coincide with the button, i.e. the user is not looking at the button, in a subsequent step 480 it is determined whether the button is in the DOWN position. If yes, the button is moved to the UP position in step 470, and the algorithm returns to the initial stage 400. If no, the button is un-primed in step 490, and the algorithm returns to the initial stage 400, ready to receive a new gaze signal.

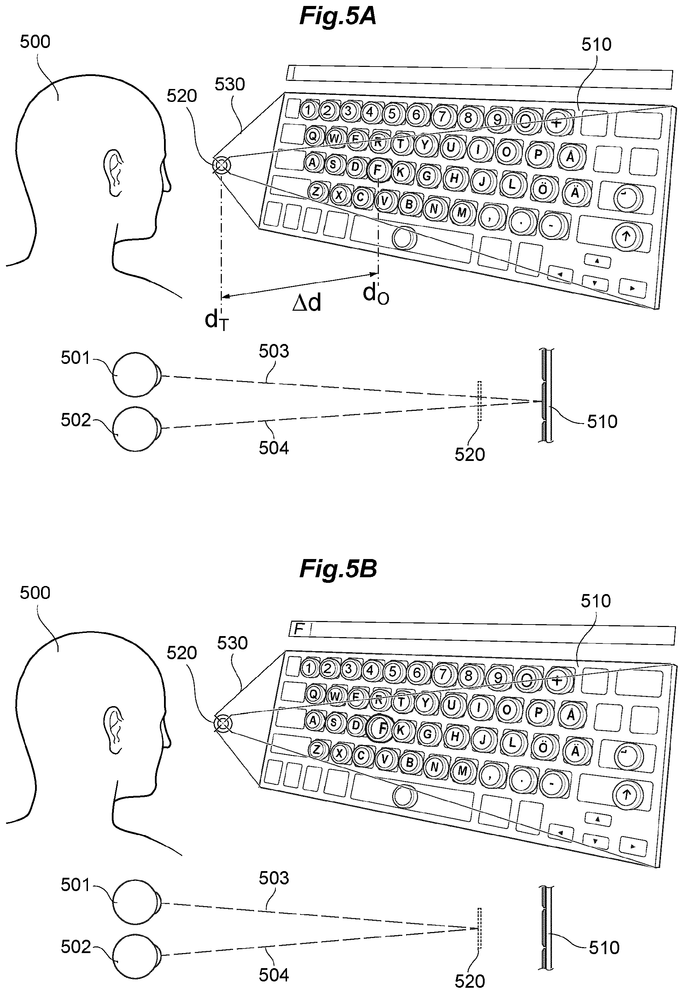

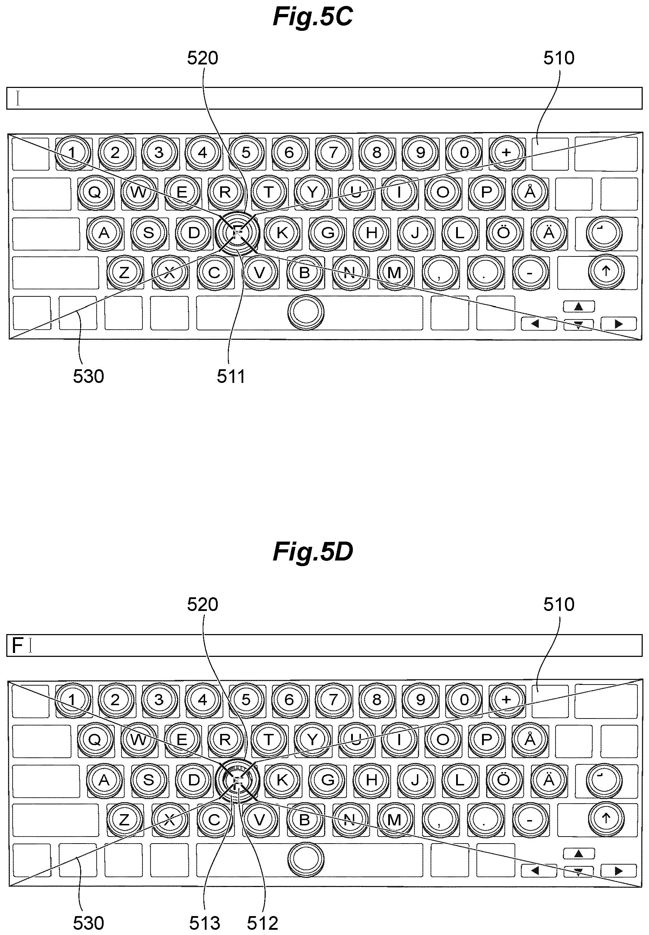

Turning now to Figs. 5A-5D, there is shown an example of a user 500 typing letters on a keyboard 510 only using the eyes by means of a method according with the present disclosure. Figs. 5A and 5B are perspective views of the user 500 and the keyboard 510 and Figs. 5C and 5D are views from the perspective of the user 500, i.e. what the user 500 sees. Initially, the user 500 scans the keyboard 510 freely looking for the first letter, e.g. 'F'.

By looking at the F-key 511, the user 500 causes it to become primed as shown in Figs. 5A and 5C. The F-key 511 grows slightly to stand out from the remaining keys, thereby providing a visual feedback to the user 500, e.g. a ghost of the F-key 511 is displayed somewhere between and in line with the F-key 511 and the user's 500 eyes 501, 502. Additionally, a trigger is displayed in the form of crosshairs 520 to further aid the user 500 in indicating both the gaze direction and the trigger distance d_{T}. In one embodiment, the position of the crosshairs 520 in relation to the keyboard 510 may be visualized to the user 500 by means of lines 530 connecting the crosshairs 520 to each of the four corners of the keyboard 510.

In the lower part of Fig. 5A, a top-down horizontal view illustrates the user's 500 gaze rays 503, 504, from the left and right eye 501, 502, respectively, focusing on the keyboard 510 displayed at the object distance d_{O} from the user 500.

The user 500 then refocuses the gaze towards crosshairs 520 displayed at the trigger distance d_{T}, i.e. the change in the convergence distance exceeds the threshold value Δd, which triggers the F-key 511 to be selected and the letter 'F' to be registered, as shown in Figs. 5B and 5D. In the lower part of Fig. 5B, a top-down horizontal view illustrates the user's 500 gaze rays 503, 504, from the left and right eye 501, 502, respectively, focusing on the crosshairs 520 displayed at the trigger distance d_{T} from the user 500.

This is further visualized in Fig. 5D wherein a central portion 512 of the F-key 511 is depressed in comparison to a surrounding ring 513, i.e. the button is DOWN in analogy with the description of the flowchart mentioned above in conjunction with Fig. 4.

In the same way that rain drops on a driver's windscreen are blurred and can be ignored while watching the road ahead, and the road ahead is blurred and can be ignored while watching the rain drops, it is possible to offer the user a more natural and less distracting representation of the invention. By taking the user's gaze convergence distance as an input to the rendered camera's focal distance on its depth of field effect, it is possible to mimic this real word effect and have a less intrusive (more transparent and blurred) and more easily located ghost.

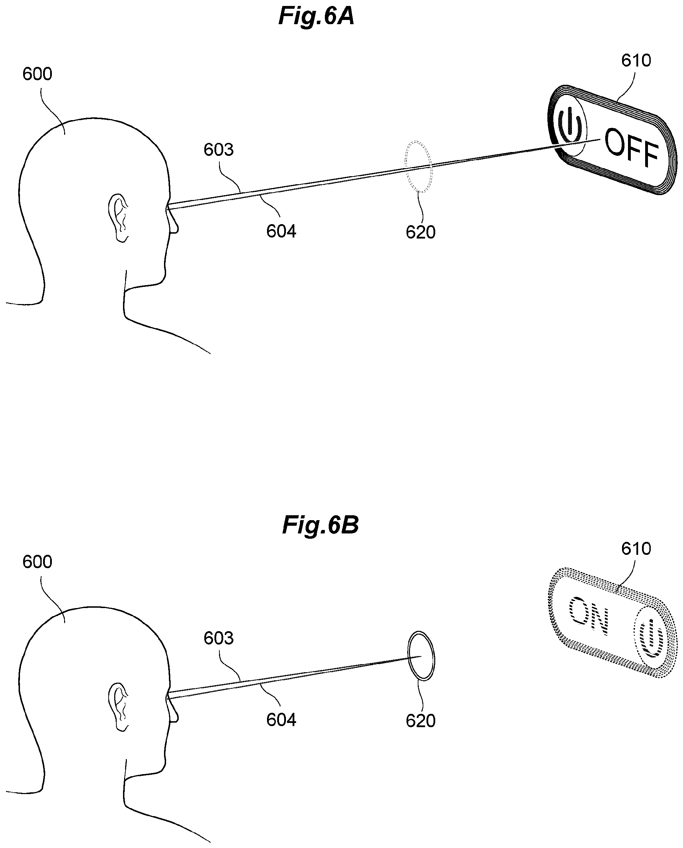

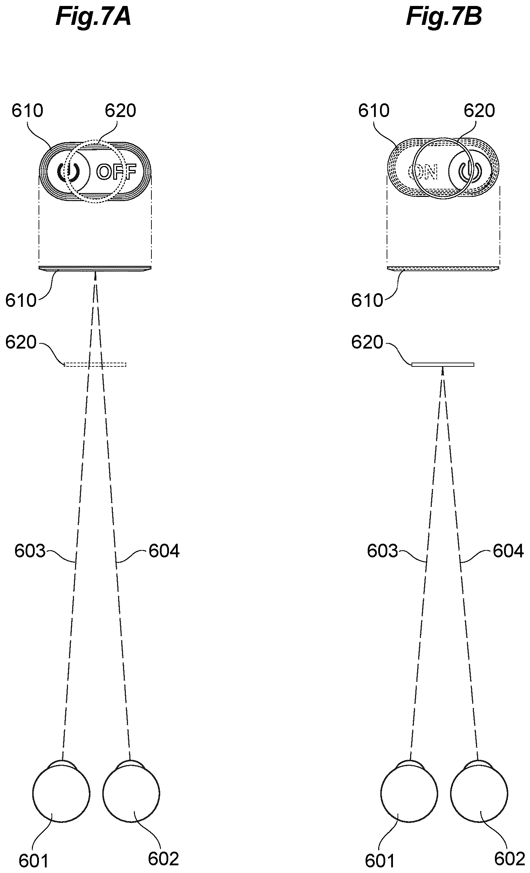

Figs. 6A-6B and 7A-7B illustrate one exemplary embodiment of implementing a differential spatial blur in a method according to the present disclosure. Figs. 6A and 6B show perspective views of the user 600 looking at a selectable object 110 in the form of an ON/OFF button 610 similar to the one shown in Figs. 3A-3C, whereas Figs. 7A and 7B show the same situations from the perspective of the user 600 combined with a top-down horizontal view similar to Figs. 1A and 1B. In Figs. 6A and 7A, the user 600 is looking at the ON/OFF button 610 in an OFF state. The convergence distance of the user 600 is at the button 610 distance corresponding to the object distance d_{O} defined above in conjunction with Figs. 1A and 1B, as indicated by the intersection of the gaze rays 603, 604 from the left and right eyes of the user 600. The observed ON/OFF button 610 is clear and in focus while the trigger in the form of a circle 620 is subjected to the depth of field effect blurring, making it more transparent and less intrusive while becoming more natural and easier to locate if needed. In Figs. 6B and 7B, the user 600 is looking at the trigger circle 620. The user's 600 gaze convergence distance is at the trigger distance d_{T}, so an interaction is effected - the ON/OFF button 610 has been toggled or switch to an ON state. The observed trigger circle 620 is clear and in focus while the ON/OFF button 610 is now subjected to the depth of field effect blurring making it more transparent and less intrusive while becoming more natural and easier to locate if needed. The effect of blurring in Figs. 6A-6B and 7A-7B is illustrated by means of dotted lines as opposed to solid lines.

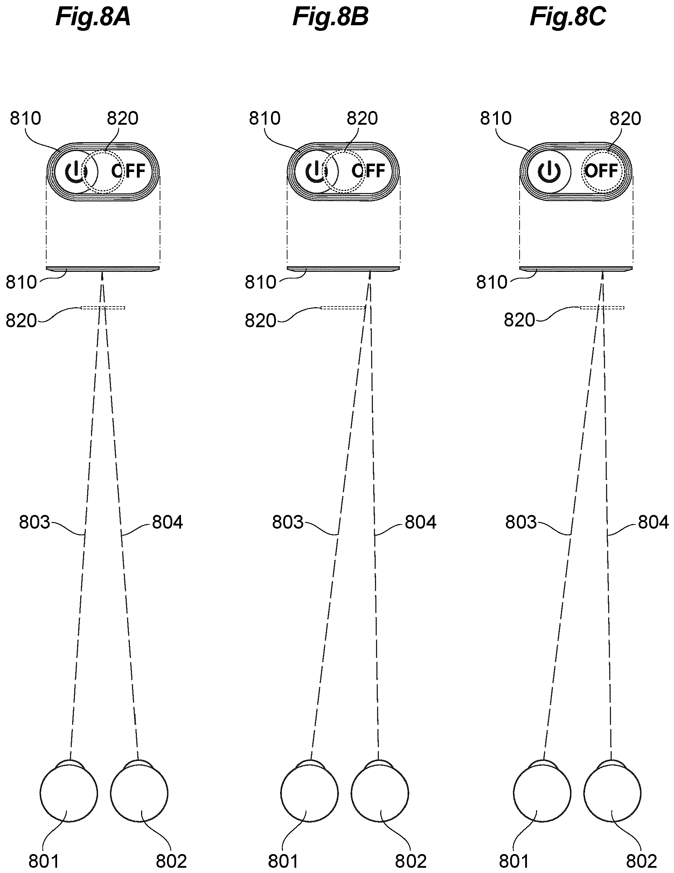

If the trigger remains perfectly aligned with the eyes 801, 802 of the user and the selectable object 810 as the user turns his head or shifts the gaze direction, the trigger 820 is more difficult to notice than if it exhibits some more physical characteristics like mass and/or damped drag. Figs. 8A-8D illustrate an additional embodiment of the present disclosure which further aids the user in locating the trigger. In Fig. 8A, the user is looking at an ON/OFF button 810 with the head stationary and the gaze rays 803, 804 centered. The ON/OFF button 810 and the trigger circle 820 are stationary and aligned with one another. In Fig. 8B, the user moves his head and/or gaze to the right. The trigger circle 820 lags behind moving momentarily out of line with the gaze rays 803, 804 from the left and right eyes 801, 802 of the user but follows the movement to the right as if attached to the head by a damped spring. In Fig. 8C, the user's head and/or gaze is again stationary with the gaze rays 803, 804 resting on the right side of the ON/OFF button 810. Now, the trigger circle 820 has caught up with the movement of the head and/or gaze and is aligned with the gaze rays 803, 804.

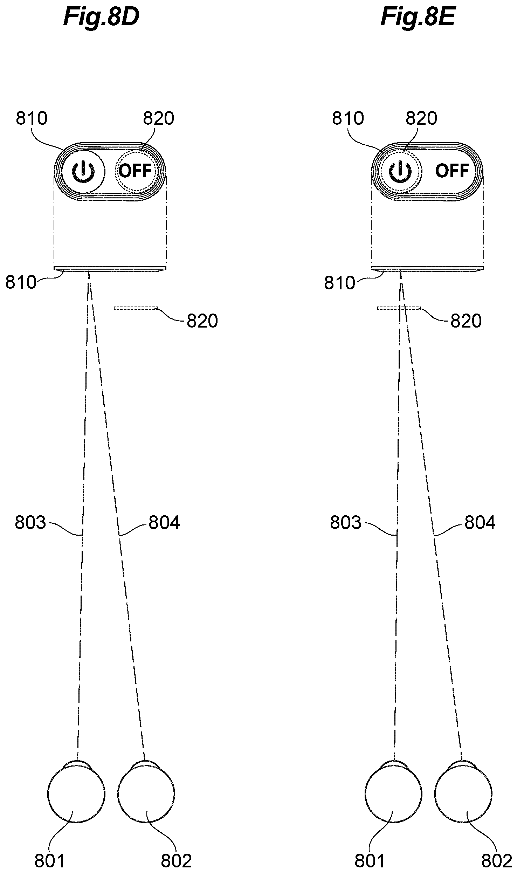

The same sequence is repeated but in the opposite direction in Figs. 8D and 8E. Fig. 8D, the user moves his head and/or gaze to the left. The trigger circle 820 lags behind moving momentarily out of line with the gaze rays 803, 804 of the user but follows the movement to the left as if attached to the head by a damped spring. In Fig. 8E, the user's head and/or gaze is again stationary with the gaze rays 803, 804 resting on the left side of the ON/OFF button 810. Now, the trigger circle 820 has caught up with the movement of the head and/or gaze and is aligned with the gaze rays 803, 804.

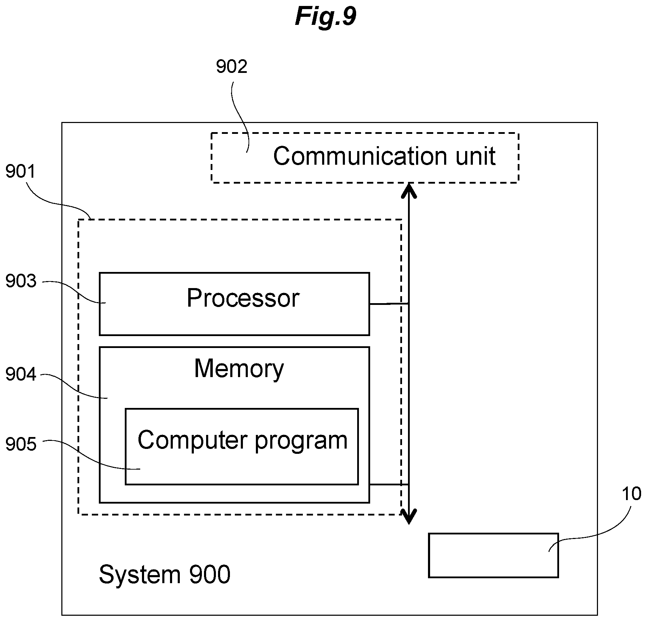

Fig. 9, in conjunction with Fig. 1, shows a system 900, capable of displaying a selectable object 110 and enabling the user to interact with the selectable object 110. The system 900 comprises a displaying device 10 arranged to display one or more selectable objects 110, processing circuitry 903, and a memory 904. The processing circuitry 903 may comprise one or more programmable processors, application-specific integrated circuits, field programmable gate arrays or combinations of these (not shown) adapted to execute instructions. The memory 904 contains instructions executable by said processing circuitry, whereby the system 900 is operative for obtaining a gaze convergence distance and a gaze direction of a user, the gaze direction lying in a field of view defined by the displaying device 10. The system 900 is further operative for determining whether the gaze direction coincides with the selectable object 110, and if so, the system 900 is further operative for detecting a change in the gaze convergence distance. The system 900 is further operative for if the detected change in the gaze convergence distance exceeds a predetermined threshold value, interacting with the selectable object 110.

According to an embodiment, the system 900 further comprises an eye tracker configured to acquire gaze data for each of the eyes of a user, wherein the system 900 is further operative for calculating the gaze convergence distance and the gaze direction based on the acquired gaze data.

According to an embodiment, the displaying device 10 is a head-mounted display (HMD) 1010 and the selectable object 110 is projected at a predetermined distance defined by the **HMD** 1010. The **HMD** 1010 may be a transparent display configured for augmented and/or mixed reality (AR/MR), or a non-transparent display configured for virtual reality, VR,.

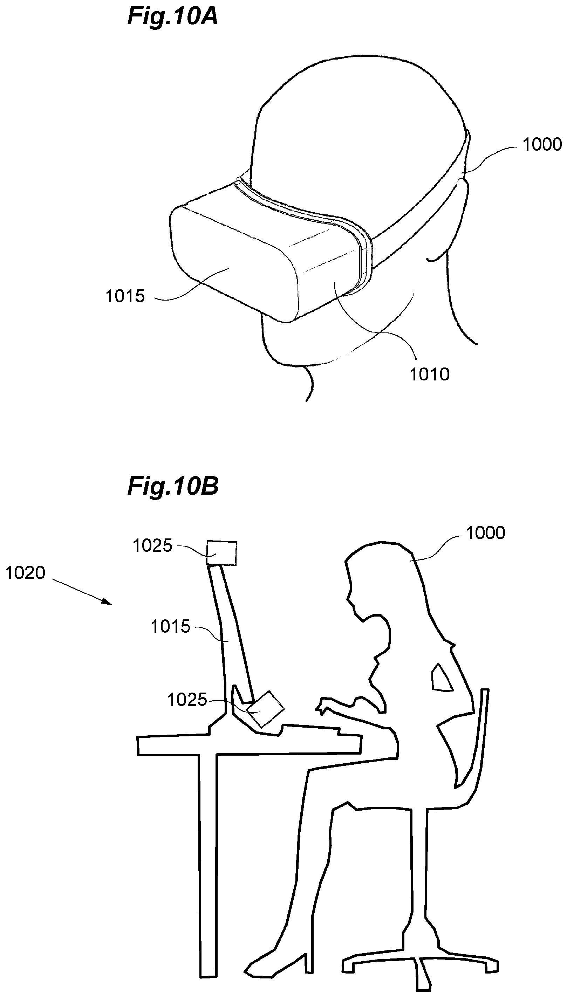

Fig. 10A shows a head-mounted device 1010 according to one or more embodiments. The head-mounted device 1010, is a device which may optionally be adapted to be mounted (or arranged) at the head of a user 1000, as shown in Fig. 10A. The head-mounted device 1010 may e.g. comprise and/or be comprised in a head-mounted display ,HMD, such as a VR headset, an AR headset or an MR headset. The head-mounted device 1010 or HMD comprises a 3D display 1015, which is able to visualize a plurality of objects 110 in response to a control signal received from a computer. The head-mounted device 1010 is typically further configured to provide eye tracker functionality by a gaze tracking signal using one or more gaze tracking sensors 1025, e.g. indicative of a gaze direction and/or a convergence distance. In other words, the head-mounted device 1010 is configured to provide an indication of an object the user is looking at and/or a depth at which the user is looking/watching. Preferably, the head-mounted device 1010 comprises one eye tracker for each eye.

The 3D display may for example be a stereoscopic display. The 3D display may for example be comprised glasses equipped with AR functionality. Further, the 3D display may be a volumetric 3D display, being either autostereoscopic or automultiscopic, which may indicate that they create 3D imagery visible to an unaided eye, without requiring stereo goggles or stereo head-mounted displays. Consequently, as described in relation to Fig.10A, the 3D display may be part of the head-mounted device 1010. However, the 3D display may also be a remote display, which does not require stereo goggles or stereo head-mounted displays. In a third example, the 3D display is a remote display, where stereoscopic glasses are needed to visualize the 3D effect to the user.

In an alternative embodiment, the displaying device 10 is a physical display such as a screen of a computer, tablet, smartphone or similar, and the selectable object 110 is displayed at the physical display.

Fig. 10B shows a remote display system 1020 according to one or more embodiments. The remote display system 1020 typically comprises a remote 3D display 1015, as described in relation to Fig. 10A. The 3D display 1015 is remote in the sense that it is not located in the immediate vicinity of the user 1000. The remote display system 1020 is typically further configured to provide eye tracker functionality by a gaze tracking signal using one or more gaze tracking sensors 1025, e.g. indicative of a gaze direction and/or a convergence distance. In other words, the remote display system 1020 is configured to provide an indication of an object the user 1000 is looking at and/or a depth at which the user is looking/watching. As can be seen from Fig. 2B, the remote 3D display 1015 does not require stereo/stereoscopic goggles or stereo/stereoscopic head-mounted displays. In a further example, the 3D display is a remote display, where stereoscopic glasses are needed to visualize the 3D effect to the user. The remote display system 1020 may comprise only one eye tracker for both eyes. In other words, the illuminator(s) and the image device(s) are arranged to illuminate/read both eyes of the user.

In some embodiments, the feature(s) of the system 900, e.g. the processing circuitry 903 and the memory 904, which perform the method steps may be a group of network nodes, wherein functionality for performing the method are spread out over different physical, or virtual, nodes of the network. In other words, the feature(s) of the system 900 which perform the method steps may be a cloud-solution, i.e. the feature(s) of the system 900 which perform the method steps may be deployed as cloud computing resources that may be distributed in the network.

According to other embodiments, the system 900 may further comprise a communication unit 902, which may be considered to comprise conventional means for communicating with relevant entities, such as other computers or devices to which it is operatively connected. The instructions executable by said processing circuitry 903 may be arranged as a computer program 905 stored e.g. in the memory 904. The processing circuitry 903 and the memory 904 may be arranged in a sub-arrangement 901. The sub-arrangement 901 may be a microprocessor and adequate software and storage therefor, a Programmable Logic Device, PLD, or other electronic component(s)/processing circuit(s) configured to perform the methods mentioned above.

The computer program 905 may comprise computer readable code means, which when run in an system 900 causes the system 900 to perform the steps described in any of the described embodiments of the system 900. The computer program 905 may be carried by a computer program product connectable to the processing circuitry 903. The computer program product may be the memory 904. The memory 904 may be realized as for example a RAM (Random-access memory), ROM (Read-Only Memory) or an EEPROM (Electrical Erasable Programmable ROM). Further, the computer program may be carried by a separate computer-readable medium, such as a CD, DVD or flash memory, from which the program could be downloaded into the memory 904. Alternatively, the computer program may be stored on a server or any other entity connected to the system 900, to which the system 900 has access via the communication unit 902. The computer program may then be downloaded from the server into the memory 904.

## Claims

1. A method for interacting with a selectable object (110) displayed by means of a displaying device (10), the method comprising the steps of:
obtaining (202), by means of an eye tracker (1025), a gaze convergence distance and a gaze direction of a user (300; 500; 600; 1000), the gaze direction lying in a field of view defined by the displaying device (10), wherein the gaze convergence distance is defined as a distance from the user to a convergence point (105, 106, 302);
determining (204) whether the gaze direction coincides with the selectable object (110); and if so,
detecting (206) a change in the gaze convergence distance; and
if the detected change in the gaze convergence distance exceeds a predetermined threshold value, Δd, interacting (208) with the selectable object (110);
interaction with the selectable object (110) is performed only if during detection of the change in the gaze convergence distance:
a predetermined change of the gaze direction is detected.

2. The method according to claim 1, wherein the step of detecting (206) further comprises:
obtaining an object distance, dₒ, indicating the distance between the user (300; 500; 600; 1000) and the selectable object (110); and
obtaining a trigger distance, dₜ,
wherein the difference between the object distance, dₒ, and the trigger distance dₜ, corresponds to the predetermined threshold value, Δd.

3. The method according to claim 2, wherein the step of detecting (206) further comprises:
priming the selectable object (110) for interaction when the gaze direction coincides with the selectable object (110) and the gaze convergence distance is within a predetermined range comprising the object distance, dₒ, and
wherein the step of interacting (208) further comprises:
interacting (208) with the primed selectable object (110).

4. The method according to claim 3, wherein priming the selectable object (110) comprises changing a displayed appearance of the selectable object (110).

5. The method according to any one of the preceding claims, further comprising:
displaying a representation (120; 320; 520; 620; 820) of the gaze direction of the user (300; 500; 600; 1000) and/or a trigger distance, dt, corresponding to the predetermined threshold value, Δd,

6. The method according to any one of the preceding claims, wherein the step of obtaining the gaze convergence distance and the gaze direction of the user (300; 500; 600; 1000) comprises acquiring gaze data for each of the eyes of the user (300; 500; 600; 1000) by means of an eye tracker (1025) and calculating the gaze convergence distance and the gaze direction based on the acquired gaze data.

7. The method according to any one of the preceding claims, wherein the gaze direction is based on a combined gaze direction, CGD, obtained by combining the gaze directions (101, 103; 301; 503, 504; 603, 604; 803, 804) of the left eye (100; 501; 601; 801) and the right eye (102; 502; 602; 802) of the user (300; 500; 600; 1000) and/or wherein the gaze convergence distance is a function of an interpupillary distance, IPD, of the user (300; 500; 600; 1000) based on the acquired pupil position or gaze ray origins of the left eye (100; 501; 601; 801) and the right eye (102; 502; 602; 802).

8. The method according to any one of the preceding claims, wherein the selectable object (110) comprises an actuatable user interface, UI, element (310; 511; 610; 810), such as a virtual switch, button, object and/or key, optionally wherein the actuatable UI element is connected to a real electronically operated switch such that interaction with the UI element causes actuation of the real electronically operated switch.

9. A system (900) for displaying and interacting with a selectable object (110), the system (900) comprising:
a displaying device (10) arranged to display one or more selectable objects 110;
processing circuitry (903);
at least one eye tracker (1025) configured to acquire gaze data for both of the eyes (100, 102; 501, 502; 601, 602; 801, 802) of a user (300; 500; 600; 1000), and
a memory (904), said memory (904) containing instructions executable by said processing circuitry (903), whereby the system (900) is operative for:
obtaining, by means of the eye tracker (1025), a gaze convergence distance and a gaze direction of a user (300; 500; 600; 1000), the gaze direction lying in a field of view defined by the displaying device (10), wherein the gaze convergence distance is defined as a distance from the user to a convergence point (105, 106, 302), wherein the system (900) is operative for calculating the gaze convergence distance and the gaze direction based on the acquired gaze data;
determining whether the gaze direction coincides with the selectable object (110); and if so,
detecting a change in the gaze convergence distance; and
if the detected change in the gaze convergence distance exceeds a predetermined threshold value, Δd, interacting with the selectable object (110);
interaction with the selectable object (110) is performed only if during detection of the change in the gaze convergence distance:
a predetermined change of the gaze direction is detected.

10. The system (900) according to claim 9, further operative for performing the method according to any one of claims 2 to 8.

11. The system (900) according to any one of claims 9-10, wherein the displaying device (10) is a head-mounted display, HMD, (1010) and the selectable object (110) is projected at the object distance, dₒ, by the HMD (1010), optionally wherein the HMD (1010) comprises a transparent or non-transparent 3D display (1015).

12. The system (900) according to any one of claims 9-10, wherein the displaying device (10) is a remote 3D display (1015) and the selectable object (110) is displayed at the remote 3D display (1015).

13. A computer program (905) comprising computer readable code means to be run in a system (900) for displaying and interacting with a selectable object (110), which computer readable code means when run in the system (900) causes the system (900) to perform the following steps:
obtaining, by means of an eye tracker (1025), a gaze convergence distance and a gaze direction of a user (300; 500; 600; 1000), the gaze direction lying in a field of view defined by the displaying device (10), wherein the gaze convergence distance is defined as a distance from the user to a convergence point (105, 106, 302);
determining whether the gaze direction coincides with the selectable object (110); and if so,
detecting a change in the gaze convergence distance; and
if the detected change in the gaze convergence distance exceeds a predetermined threshold value, Δd, interacting with the selectable object (110);
interaction with the selectable object (110) is performed only if during detection of the change in the convergence distance:
a predetermined change of the gaze direction is detected.

14. A carrier containing the computer program (905) according to claim 13, wherein the carrier is one of an electronic signal, an optical signal, a radio signal or a computer readable storage medium.

## Patentansprüche

1. Verfahren zum Zusammenwirken mit einem auswählbaren Objekt (110), das mittels einer Anzeigevorrichtung (10) angezeigt wird, das Verfahren umfassend die Schritte:
Erhalten (202), mittels einer Augenverfolgungseinrichtung (1025), eines Blickkonvergenzabstands und einer Blickrichtung eines Benutzers (300; 500; 600; 1000), wobei die Blickrichtung in einem durch die Anzeigevorrichtung (10) definierten Sichtfeld liegt, wobei der Blickkonvergenzabstand als ein Abstand von dem Benutzer zu einem Konvergenzpunkt (105, 106, 302) definiert ist;
Bestimmen (204), ob die Blickrichtung mit dem auswählbaren Objekt (110) übereinstimmt; und falls ja,
Erkennen (206) einer Änderung des Blickkonvergenzabstands; und
falls die erkannte Änderung des Blickkonvergenzabstands einen vorbestimmten Grenzwert, Δd, überschreitet, Zusammenwirken (208) mit dem auswählbaren Objekt (110);
wobei das Zusammenwirken mit dem auswählbaren Objekt (110) nur durchgeführt wird, falls während der Erkennung der Änderung des Blickkonvergenzabstands:
eine vorbestimmte Änderung der Blickrichtung erkannt wird.

2. Verfahren nach Anspruch 1, wobei der Schritt des Erkennens (206) umfasst:
Erhalten eines Objektabstands, d₀, der den Abstand zwischen dem Benutzer (300; 500; 600; 1000) und dem auswählbaren Objekt (110) angibt; und
Erhalten eines Auslöseabstands, dt, wobei die Differenz zwischen dem Objektabstand, d₀, und dem Auslöseabstand dt dem vorbestimmten Grenzwert, Δd, entspricht.

3. Verfahren nach Anspruch 2, wobei der Schritt des Erkennens (206) ferner umfasst:
Vorbereiten des auswählbaren Objekts (110) zum Zusammenwirken, wenn die Blickrichtung mit dem auswählbaren Objekt (110) übereinstimmt und der Blickkonvergenzabstand innerhalb eines vorbestimmten Bereichs, umfassend den Objektabstand, d₀, liegt, und
wobei der Schritt des Zusammenwirkens (208) ferner umfasst:
Zusammenwirken (208) mit dem vorbereiteten auswählbaren Objekt (110).

4. Verfahren nach Anspruch 3, wobei das Vorbereiten des auswählbaren Objekts (110) das Ändern eines angezeigten Erscheinungsbilds des auswählbaren Objekts (110) umfasst.

5. Verfahren nach einem der vorstehenden Ansprüche, ferner umfassend:
Anzeigen einer Darstellung (120; 320; 520; 620; 820) der Blickrichtung des Benutzers (300; 500; 600; 1000) und/oder eines Auslöseabstands, dt, entsprechend dem vorbestimmten Grenzwert, Δd,

6. Verfahren nach einem der vorstehenden Ansprüche, wobei der Schritt des Erhaltens des Blickkonvergenzabstands und der Blickrichtung des Benutzers (300; 500; 600; 1000) ein Erfassen von Blickdaten für jedes der Augen des Benutzers (300; 500; 600; 1000) mittels einer Augenverfolgungseinrichtung (1025) und ein Berechnen des Blickkonvergenzabstands und der Blickrichtung basierend auf den erfassten Blickdaten umfasst.

7. Verfahren nach einem der vorstehenden Ansprüche, wobei die Blickrichtung auf einer kombinierten Blickrichtung, CGD, basiert, die durch Kombinieren der Blickrichtungen (101, 103; 301; 503, 504; 603, 604; 803, 804) des linken Auges (100; 501; 601; 801) und des rechten Auges (102; 502; 602; 802) des Benutzers (300; 500; 600; 1000) erhalten wird, und/oder wobei der Blickkonvergenzabstand von einem Pupillenabstand, IPD, des Benutzers (300; 500; 600; 1000) abhängig ist, basierend auf der erfassten Pupillenposition oder den Blickstrahlursprüngen des linken Auges (100; 501; 601; 801) und des rechten Auges (102; 502; 602; 802).

8. Verfahren nach einem der vorstehenden Ansprüche, wobei das auswählbare Objekt (110) ein betätigbares Benutzerschnittstellenelement, **UI-**Element (310; 511; 610; 810) umfasst, wie einen virtuellen Schalter, eine Schaltfläche, ein Objekt und/oder eine Taste, wobei optional das betätigbare **UI-**Element mit einem realen elektronisch betriebenen Schalter derart verbunden ist, dass das Zusammenwirken mit dem UI-Element eine Betätigung des realen elektronisch betriebenen Schalters bewirkt.

9. System (900) zum Anzeigen und Zusammenwirken mit einem auswählbaren Objekt (110), das System (900) umfassend:
eine Anzeigevorrichtung (10), die angeordnet ist, um ein oder mehrere auswählbare Objekte 110 anzuzeigen;
eine Verarbeitungsschaltung (903);
mindestens eine Augenverfolgungseinrichtung (1025), die konfiguriert ist, um Blickdaten für beide der Augen (100, 102; 501, 502; 601, 602; 801, 802) eines Benutzers (300; 500; 600; 1000) zu erfassen, und
einen Speicher (904), wobei der Speicher (904) Anweisungen enthält, die durch die Verarbeitungsschaltung (903) ausführbar sind, wobei das System (900) betreibbar ist zum:
Erhalten, mittels der Augenverfolgungseinrichtung (1025), eines Blickkonvergenzabstands und einer Blickrichtung eines Benutzers (300; 500; 600; 1000), wobei die Blickrichtung in einem durch die Anzeigevorrichtung (10) definierten Sichtfeld liegt, wobei der Blickkonvergenzabstand als ein Abstand von dem Benutzer zu einem Konvergenzpunkt (105, 106, 302) definiert ist, wobei das System (900) zum Berechnen des Blickkonvergenzabstands und der Blickrichtung basierend auf den erfassten Blickdaten betreibbar ist;
Bestimmen, ob die Blickrichtung mit dem auswählbaren Objekt (110) übereinstimmt; und falls ja,
Erkennen einer Änderung des Blickkonvergenzabstands; und
falls die erkannte Änderung des Blickkonvergenzabstands einen vorbestimmten Grenzwert, Δd, überschreitet, Zusammenwirken mit dem auswählbaren Objekt (110);
wobei das Zusammenwirken mit dem auswählbaren Objekt (110) nur durchgeführt wird, falls während der Erkennung der Änderung des Blickkonvergenzabstands:
eine vorbestimmte Änderung der Blickrichtung erkannt wird.

10. System (900) nach Anspruch 9, das ferner zum Durchführen des Verfahrens nach einem der Ansprüche 2 bis 8 betreibbar ist.

11. System (900) nach einem der Ansprüche 9 bis 10, wobei die Anzeigevorrichtung (10) ein Datenhelm, **HMD,** (1010) ist und das auswählbare Objekt (110) in dem Objektabstand, d₀, durch den **HMD** (1010) projiziert wird, optional wobei der **HMD** (1010) eine transparente oder nicht-transparente 3D-Anzeige (1015) umfasst.

12. System (900) nach einem der Ansprüche 9 bis 10, wobei die Anzeigevorrichtung (10) eine entfernte 3D-Anzeige (1015) ist und das auswählbare Objekt (110) auf der entfernten 3D-Anzeige (1015) angezeigt wird.

13. Computerprogramm (905), umfassend computerlesbare Codemittel, die in einem System (900) zum Anzeigen und Zusammenwirken mit einem auswählbaren Objekt (110) laufen gelassen werden sollen, wobei die computerlesbaren Codemittel, wenn sie in dem System (900) laufen gelassen werden, das System (900) veranlassen, die folgenden Schritte durchzuführen:
Erhalten, mittels einer Augenverfolgungseinrichtung (1025), eines Blickkonvergenzabstands und einer Blickrichtung eines Benutzers (300; 500; 600; 1000), wobei die Blickrichtung in einem durch die Anzeigevorrichtung (10) definierten Sichtfeld liegt, wobei der Blickkonvergenzabstand als ein Abstand von dem Benutzer zu einem Konvergenzpunkt (105, 106, 302) definiert ist;
Bestimmen, ob die Blickrichtung mit dem auswählbaren Objekt (110) übereinstimmt; und falls ja,
Erkennen einer Änderung des Blickkonvergenzabstands; und
falls die erkannte Änderung des Blickkonvergenzabstands einen vorbestimmten Grenzwert, Δd, überschreitet, Zusammenwirken mit dem auswählbaren Objekt (110);
wobei das Zusammenwirken mit dem auswählbaren Objekt (110) nur durchgeführt wird, falls während der Erkennung der Änderung des Konvergenzabstands:
eine vorbestimmte Änderung der Blickrichtung erkannt wird.

14. Träger, der das Computerprogramm (905) nach Anspruch 13 enthält, wobei der Träger eines von einem elektronischen Signal, einem optischen Signal, einem Funksignal oder einem computerlesbaren Speicherungsmedium ist.

## Revendications

1. Procédé permettant d'interagir avec un objet sélectionnable (110) affiché au moyen d'un dispositif d'affichage (10), le procédé comprenant les étapes consistant à :
obtenir (202), au moyen d'un oculomètre (1025), une distance de convergence de regard et une direction de regard d'un utilisateur (300 ; 500 ; 600 ; 1000), la direction de regard se trouvant dans un champ de vision défini par le dispositif d'affichage (10), dans lequel la distance de convergence de regard est définie comme une distance de l'utilisateur à un point de convergence (105, 106, 302) ;
déterminer (204) si la direction de regard coïncide avec l'objet sélectionnable (110) ; et si oui,
détecter (206) un changement dans la distance de convergence de regard ; et
si le changement détecté dans la distance de convergence de regard dépasse une valeur seuil prédéterminée, Δd, interagir (208) avec l'objet sélectionnable (110) ;
l'interaction avec l'objet sélectionnable (110) est réalisée uniquement si pendant la détection du changement dans la distance de convergence de regard :
un changement prédéterminé de la direction de regard est détecté.

2. Procédé selon la revendication 1, dans lequel l'étape consistant à détecter (206) comprend en outre :
l'obtention d'une distance d'objet, d₀, indiquant la distance entre l'utilisateur (300 ; 500 ; 600 ; 1000) et l'objet sélectionnable (110) ; et
l'obtention d'une distance de déclenchement, dt, dans lequel la différence entre la distance d'objet, d₀, et la distance de déclenchement dt, correspond à la valeur seuil prédéterminée, Δd.

3. Procédé selon la revendication 2, dans lequel l'étape consistant à détecter (206) comprend en outre :
l'amorçage de l'objet sélectionnable (110) pour une interaction lorsque la direction de regard coïncide avec l'objet sélectionnable (110) et que la distance de convergence de regard est au sein d'une plage prédéterminée comprenant la distance d'objet, d₀, et
dans lequel l'étape consistant à interagir (208) comprend en outre :
l'interaction (208) avec l'objet sélectionnable amorcé (110).

4. Procédé selon la revendication 3, dans lequel l'amorçage de l'objet sélectionnable (110) comprend le changement d'une apparence affichée de l'objet sélectionnable (110).

5. Procédé selon l'une quelconque des revendications précédentes, comprenant en outre :
l'affichage d'une représentation (120 ; 320 ; 520 ; 620 ; 820) de la direction de regard de l'utilisateur (300 ; 500 ; 600 ; 1000) et/ou d'une distance de déclenchement, dt, correspondant à la valeur seuil prédéterminée, Δd,

6. Procédé selon l'une quelconque des revendications précédentes, dans lequel l'étape consistant à obtenir la distance de convergence de regard et la direction de regard de l'utilisateur (300 ; 500 ; 600 ; 1000) comprend l'acquisition de données de regard pour chacun des yeux de l'utilisateur (300 ; 500 ; 600 ; 1000) au moyen d'un oculomètre (1025) et le calcul de la distance de convergence de regard et de la direction de regard sur la base des données de regard acquises.

7. Procédé selon l'une quelconque des revendications précédentes, dans lequel la direction de regard est en fonction d'une direction de regard combinée, CGD, obtenue par la combinaison des directions de regard (101, 103 ; 301 ; 503, 504 ; 603, 604 ; 803, 804) de l'œil gauche (100 ; 501 ; 601 ; 801) et de l'œil droit (102 ; 502 ; 602 ; 802) de l'utilisateur (300 ; 500 ; 600 ; 1000) et/ou dans lequel la distance de convergence de regard est une fonction d'une distance interpupillaire, IPD, de l'utilisateur (300 ; 500 ; 600 ; 1000) sur la base de la position de pupille acquise ou des origines de rayon de regard de l'œil gauche (100 ; 501 ; 601 ; 801) et de l'oeil droit (102 ; 502 ; 602 ; 802).

8. Procédé selon l'une quelconque des revendications précédentes, dans lequel l'objet sélectionnable (110) comprend un élément d'interface utilisateur, UI, actionnable (310 ; 511 ; 610 ; 810), tel qu'un commutateur, un bouton, un objet et/ou une touche virtuels, éventuellement dans lequel l'élément d'UI actionnable est connecté à un commutateur à fonctionnement électronique réel de telle sorte qu'une interaction avec l'élément d'UI amène l'actionnement du commutateur à fonctionnement électronique réel.

9. Système (900) permettant d'afficher et d'interagir avec un objet sélectionnable (110), le système (900) comprenant :
un dispositif d'affichage (10) agencé pour afficher un ou plusieurs objets sélectionnables 110 ;
une circuiterie de traitement (903) ;
au moins un oculomètre (1025) configuré pour acquérir des données de regard pour les deux yeux (100, 102 ; 501, 502 ; 601, 602 ; 801, 802) d'un utilisateur (300 ; 500 ; 600 ; 1000), et
une mémoire (904), ladite mémoire (904) contenant des instructions exécutables par ladite circuiterie de traitement (903), moyennant quoi le système (900) est fonctionnel pour :
obtenir, au moyen de l'oculomètre (1025), une distance de convergence de regard et une direction de regard d'un utilisateur (300 ; 500 ; 600 ; 1000), la direction de regard se trouvant dans un champ de vision défini par le dispositif d'affichage (10), dans lequel la distance de convergence de regard est définie comme une distance de l'utilisateur à un point de convergence (105, 106, 302), dans lequel le système (900) est fonctionnel pour calculer la distance de convergence de regard et la direction de regard sur la base des données de regard acquises ;
déterminer si la direction de regard coïncide avec l'objet sélectionnable (110) ; et si oui,
détecter un changement dans la distance de convergence de regard ; et
si le changement détecté dans la distance de convergence de regard dépasse une valeur seuil prédéterminée, Δd, interagir avec l'objet sélectionnable (110) ;
l'interaction avec l'objet sélectionnable (110) est réalisée uniquement si pendant la détection du changement dans la distance de convergence de regard :
un changement prédéterminé de la direction de regard est détecté.

10. Système (900) selon la revendication 9, fonctionnel en outre pour la réalisation du procédé selon l'une quelconque des revendications 2 à 8.

11. Système (900) selon l'une quelconque des revendications 9 à 10, dans lequel le dispositif d'affichage (10) est un visiocasque, **HMD,** (1010) et l'objet sélectionnable (110) est projeté à la distance d'objet, d₀, par le **HMD** (1010), éventuellement dans lequel le **HMD** (1010) comprend un affichage 3D (1015) transparent ou non transparent.

12. Système (900) selon l'une quelconque des revendications 9 à 10, dans lequel le dispositif d'affichage (10) est un affichage 3D (1015) distant et l'objet sélectionnable (110) est affiché au niveau de l'affichage 3D (1015) distant.

13. Programme d'ordinateur (905) comprenant un moyen de code lisible par ordinateur destiné à être exécuté dans un système (900) pour l'affichage et l'interaction avec un objet sélectionnable (110), lequel moyen de code lisible par ordinateur lorsqu'il est exécuté dans le système (900) amène le système (900) à réaliser les étapes suivantes :
obtenir, au moyen d'un oculomètre (1025), une distance de convergence de regard et une direction de regard d'un utilisateur (300 ; 500 ; 600 ; 1000), la direction de regard se trouvant dans un champ de vision défini par le dispositif d'affichage (10), dans lequel la distance de convergence de regard est définie comme une distance de l'utilisateur à un point de convergence (105, 106, 302) ;
déterminer si la direction de regard coïncide avec l'objet sélectionnable (110) ; et si oui,
détecter un changement dans la distance de convergence de regard ; et
si le changement détecté dans la distance de convergence de regard dépasse une valeur seuil prédéterminée, Δd, interagir avec l'objet sélectionnable (110) ;
l'interaction avec l'objet sélectionnable (110) est réalisée uniquement si pendant la détection du changement dans la distance de convergence :
un changement prédéterminé de la direction de regard est détecté.

14. Porteuse contenant le programme d'ordinateur (905) selon la revendication 13, dans laquelle la porteuse est l'un parmi un signal électronique, un signal optique, un signal radio ou un support de stockage lisible par ordinateur.
